Europäisches Patentamt

European Patent Office

Office européen des brevets

(19)

(11) Veröffentlichungsnummer: **0 225 850**
**B1**

# (12) EUROPÄISCHE PATENTSCHRIFT

(45) Veröffentlichungstag der Patentschrift:
28.02.90

(21) Anmeldenummer: 86810554.5

(22) Anmeldetag: 01.12.86

(51) Int. Cl.⁴: **C07D 211/46, C08K 5/34**

(54) **Verfahren zur Herstellung von 1-(2-Hydroxyethyl)-2,2,6,6-tetramethyl-4-hydroxy-piperidin.**

(30) Priorität: 06.12.85 CH 5226/85

(43) Veröffentlichungstag der Anmeldung:
16.06.87 Patentblatt 87/25

(45) Bekanntmachung des Hinweises auf die Patenterteilung:
28.02.90 Patentblatt 90/9

(84) Benannte Vertragsstaaten:
BE CH DE FR GB IT LI

(56) Entgegenhaltungen:
EP-A- 0 119 558
DE-A- 2 656 764
DE-A- 2 656 765
JP-A- 5 721 368

**Die Akte enthält technische Angaben, die nach dem Eingang der Anmeldung eingereicht wurden und die nicht in dieser Patentschrift enthalten sind.**

(73) Patentinhaber: CIBA-GEIGY AG, Klybeckstrasse 141,
CH-4002 Basel(CH)

(72) Erfinder: Issler, Heinz, Dr., Weinbergstrasse 20,
D-6140 Bensheim(DE)
Erfinder: Kintopf, Siegfried, Dr., Blütenweg 22,
D-6140 Bensheim/Bergstrasse(DE)
Erfinder: König, Lutz, Dr., Juttastrasse 91,
D-6520 Worms 15(DE)
Erfinder: Stephan, Hans, Jakobsweg 89,
D-6140 Bensheim(DE)

## Beschreibung

Die vorliegende Erfindung betrifft ein Verfahren zur Herstellung von 1-(2-Hydroxyethyl)-2,2,6,6-tetramethyl-4-hydroxy-piperidin, im folgenden auch HE-HTMP genannt, durch katalytische Reduktion von 2,2,6,6-Tetramethyl-4-oxo-piperidin, auch Triacetonamin genannt und im folgenden mit TAA abgekürzt, und anschliessende Umsetzung des entstandenen 2,2,6,6-Tetramethyl-4-hydroxy-piperidins (HTMP) mit Ethylenoxid.

HE-HTMP ist ein nützlicher Licht- und Wärmestabilisator für Kunststoffe und ausserdem ein wertvolles Zwischenprodukt für die Synthese von Lichtschutz-Additiven.

Die Synthese von HE-HTMP erfolgt im allgemeinen ausgehend von TAA in zwei Schritten, wobei TAA zunächst mittels katalytischer Hydrierung z.B. gemäss der CH-A 602 644 zu HTMP reduziert wird. Die weitere Umsetzung von HTMP zu HE-HTMP ist z.B. aus der JP-A 57-21368 bekannt, wobei dort ein grosser und wenig wünschenswerter Ethylenoxid-Ueberschuss benutzt wird. Die Synthese von HE-HTMP aus HTMP und Ethylendoxid in Gegenwart eines stark basischen Katalysators wird z.B. in den US-A 3 974 127 und 4 001 190 beschrieben, wobei das Verfahren eine komplizierte und für das Arbeiten im technischen Massstab unvorteilhafte Temperaturführung aufweist.

Der hauptsächliche Nachteil der bekannten HE-HTMP-Synthese-Verfahren besteht darin, dass das Zwischenprodukt, HTMP, bisher vor der Weiterreaktion grundsätzlich z.B. mittels Aussalzens oder Umkristallisierens isoliert bzw. gereinigt werden muss, wenn HE-HTMP in einer akzeptablen Ausbeute erhalten werden soll. Die Zwischenisolierung des HTMP bringt jedoch Ausbeute-Verluste mit sich, ist zeitaufwendig und belastet die Umwelt.

Es wurde nun gefunden, dass HE-HTMP aus TAA unter Vermeidung der angesprochenen Nachteile, insbesondere ohne Zwischenisolierung des HTMP, erhalten werden kann, wenn man die bei der katalytischen Hydrierung anfallende HTMP-Rohlösung vor der Weiterreaktion mit einer katalytischen Menge Säure versetzt und gegebenenfalls durch Destillation aufkonzentriert.

Die Erfindung betrifft daher ein Verfahren zur Herstellung von HE-HTMP aus TAA, dadurch gekennzeichnet, dass man TAA in an sich bekannter Weise mittels katalytischer Hydrierung in Wasser oder in polaren organischen Lösungsmitteln oder in deren Mischungen mit Wasser zu 2,2,6,6-Tetramethyl-4-hydroxy-piperidin (HTMP) reduziert, die anfallende HTMP-Rohlösung, als solche oder nach deren Aufkonzentrierung durch Destillation, ohne weitere Reinigung und/oder Zwischenisolierung des HTMP mit einer katalytischen Menge einer anorganischen oder organischen Säure versetzt, wobei im Falle der Aufkonzentrierung die Säurezugabe auch vor der Destillation erfolgen kann, und wobei die aufkonzentrierte Rohlösung bzw. Rohschmelze gegebenenfalls mit dem gleichen oder einem anderen Lösungsmittel wieder verdünnt werden kann, und anschliessend mit Ethylenoxid in einem molaren Ueberschuss von 1-35 % bei einer Temperatur von 60-170°C umsetzt.

Die erste Stufe (katalytische Hydrierung von TAA) wird in wässriger Lösung, in polaren organischen Lösungsmitteln oder in deren Mischungen mit Wasser durchgeführt. Werden polare organische Lösungsmittel eingesetzt, sind dies vorzugsweise solche, die mit Wasser mischbar sind. Als derartige Lösungsmittel kommen z.B. ein- oder mehrwertige Alkohole mit 1 bis 5 C-Atomen oder Etheralkohole in Betracht, wie etwa einwertige Alkohole mit 1 bis 5 C-Atomen, Glykole und Glykolether. Beispiele hierfür sind Methanol, Ethanol, Isopropanol, n-Propanol, Butanol, Ethylenglykol, Ethylenglykolether usw. In einer Ausführungsform werden z.B. die genannten polaren organischen Lösungsmittel verwendet, in einer anderen Mischungen solcher Lösungsmittel mit Wasser. Bevorzugt wird die katalytische Hydrierung von TAA jedoch in wässriger Lösung durchgeführt.

In einer weiteren Ausführungsform kann neben den vorstehend genannten Lösungsmitteln (einschliesslich Wasser) auch noch ein nicht-polares organisches Lösungsmittel mitverwendet werden. Als derartige Lösungsmittel kommen z.B. aliphatische oder aromatische Kohlenwasserstoffe in Frage, z.B. Benzol, Toluol, Xylol usw.

Die Hydrierung kann in an sich bekannter Weise unter Verwendung von üblichen Hydrierkatalysatoren, insbesondere Edelmetallkatalysatoren durchgeführt werden. Als solche seien insbesondere Raney-Nickel und vor allem Rutheniumkohle genannt. Bevorzugt wird die Hydrierung in der in der CH-A 602 644 beschriebenen Weise durchgeführt.

Die nach der Hydrierung erhaltene HTMP-Rohlösung kann direkt als solche weiterverwendet, d.h. nach Zugabe von Säure mit Ethylenoxid versetzt werden. Oft ist es jedoch zweckmässig, das Volumen der Rohlösung vor der Weiterverarbeitung zu reduzieren. Dies geschieht durch Destillation bei Normaldruck oder unter vermindertem Druck. In einer Ausführungsform des erfindungsgemässen Verfahrens wird daher die Rohlösung bis zu einem HTMP-Gehalt von mindestens 30 % (z.B. 30 bis 60 %), vorzugsweise mindestens 50 %, durch Destillation aufkonzentriert. Beispielsweise wird bis zu einem HTMP-Gehalt von 70 % oder 80 % aufkonzentriert. Sehr gute Ergebnisse werden auch erhalten, wenn die Aufkonzentrierung bis zu einem HTMP-Gehalt von 90 % oder bis zu einer HTMP-Rohschmelze weitergeführt wird, als wenn praktisch das gesamte Lösungsmittel abdestilliert wird. Dies bedeutet jedoch keine Isolierung des HTMP, da sich das Lösungsmittel auf diese Weise nicht ganz entfernen lässt und Nebenprodukte ebenfalls noch in der Rohschmelze vorhanden sind.

Wird eine destillative Behandlung vorgenommen, destilliert man die HTMP-Rohlösung bei Normaldruck oder vorzugsweise vermindertem Druck, wobei ein verminderter Druck von 100 bis 900 mbar und insbesondere 100 bis 300 mbar zweckmässig ist. Hierbei geht bei einer Temperaturvon 30° bis 100°C je nach gewähltem Druck ein zweiphasiges, aus einer wässrigen und einer öligen Phase bestehendes Gemisch über, welches verworfen wird.

Wird die HTMP-Rohlösung destilliert, kann die Säurezugabe im Prinzip vor der Destillation oder nach einer solchen erfolgen. Vorzugsweise gibt man jedoch die Säure nach der Destillation der Rohlösung bzw. -schmelze zu.

Wird die aus der Hydrierstufe erhaltene Rohlösung destilliert, kann die verbleibende konzentrierte Lösung bzw. Schmelze vor der Weiterverarbeitung wieder verdünnt werden, wobei das gleiche oder ein anderes Lösungsmittel verwendet werden kann. Es können hierzu beispielsweise die oben für die Hydrierstufe angegebenen Lösungsmittel (einschliesslich Wasser und der gegebenenfalls mitzuverwendenden unpolaren Lösungsmittel) eingesetzt werden. Die Wahl des Lösungsmittels kann sich danach richten, auf welche Weise man das Endprodukt HE-HTMP zu isolieren wünscht bzw. ob es für eine eventuelle Weiterverarbeitung gleich im dort benötigten Lösungsmittel anfallen soll.

Bei der in katalytischen Mengen zuzugebenden Säure kann es sich um eine beliebige, vorzugsweise protonenhaltige, anorganische oder organische Säure handeln. Beispiele für geeignete Säuren sind anorganische Säuren wie etwa HCl, HBr, $H_2SO_4$, $H_3PO_4$, $H_2SO_3$ und $HNO_3$, und organische aromatische oder aliphatische Carbonsäuren wie etwa Ameisensäure, Essigsäure, Halogenessigsäuren, Propionsäure, Benzoesäure und substituierte Benzoesäuren sowie aromatische Sulfonsäuren wie etwa Benzolsulfonsäure und substituierte Benzolsulfonsäuren, z.B. p-Toluolsulfonsäure. Bevorzugt ist die Verwendung von HCl, Ameisensäure, Essigsäure, p-Toluolsulfonsäure insbesondere $H_2SO_4$.

Die anorganische oder organische Säure wird bevorzugt in einer Menge von 0,1 bis 10 %, z.B. 0,1 bis 5 %, insbesondere 0,3 bis 3 % und besonders bevorzugt 0,5 bis 1 %, bezogen auf die gegebenenfalls aufkonzentrierte und gegebenenfalls wieder verdünnte HTMP-Rohlösung bzw. -Rohschmelze, zugegeben.

Vorzugsweise verwendet man 0,5 bis 1,0 % $H_2SO_4$, bezogen auf die gegebenenfalls aufkonzentrierte HTMP-Rohlösung bzw. -Rohschmelze, als katalytische Menge Säure.

Das Ethylenoxid wird in einem molaren Ueberschuss von 1 bis 35, z.B. 10-35 %, bezogen auf das in Lösung befindliche HTMP, eingesetzt. Die Zugabe des Ethylenoxids zur Reaktionslösung erfolgt bevorzugt innerhalb von 1 bis 120 min und besonders bevorzugt innerhalb von 1 bis 40 min, z.B. 1 bis 10 min.

Die Umsetzung mit Ethylenoxid findet im Temperaturbereich von 60-170°C, z.B. 60-120°C, statt. Es ist zweckmässig, die Reaktion in einem Autoklaven durchzuführen. Dabei wird das Ethylenoxid vorzugsweise bei einer bestimmten Temperatur zugesetzt und die Reaktion läuft dann ohne externe Kühlung oder Erwärmung ab ("quasi-adiabatische Verfahrensführung"). Beispielsweise erfolgt die Ethylenoxidzugabe bei Temperaturen von 80-100°C, worauf die Temperatur in der Regel ansteigt, z.B. bis etwa 120°C. Es kann aber auch ein Temperaturrückgang eintreten. In manchen Fällen ist es zweckmässig, eine Nachreaktionszeit einzuschalten, wobei gegebenenfalls auch erwärmt werden kann.

Nach beendeter Reaktion kann das Reaktionsgemisch in üblicher Weise aufgearbeitet werden. So kann das Endprodukt durch Abkühlung oder Zusatz von geeigneten Fällungsmitteln ausgefällt, abfiltriert, gegebenenfalls gewaschen und umkristallisiert werden. Wenn eine Weiterverarbeitung erfolgt, kann auch die erhaltene Lösung direkt eingesetzt werden.

Das erfindungsgemässe Verfahren zur Herstellung von HE-HTMP aus TAA kann diskontinuierlich (batchwise) oder kontinuierlich, z.B. in einem Rotating Disc-Reaktor oder in einer Reaktionskaskade ausgeführt werden. Hierbei ist eine kontinuierliche Verfahrensweise bevorzugt.

In einer erwähnenswerten Ausführungsform des erfindungsgemässen Verfahrens führt man die katalytische Hydrierung des TAA in wässriger Lösung durch und setzt die erhaltene Rohlösung, gegebenenfalls nach destillativer Aufkonzentrierung auf einen HTMP-Gehalt von bis zu 70 %, mit Ethylenoxid bei 60-120°C unter quasi-adiabatischer Verfahrensführung um, wobei ein molarer Ethylenoxidüberschuss von 10-35 % verwendet wird und das erhaltene Produkt in bekannter Weise isoliert und aufarbeitet.

Für die Bestimmung des Gehaltes an HTMP (Rohlösung) und HE-HTMP (gegebenenfalls anfallende Endprodukt-Lösung) können übliche analytische Methoden verwendet werden, wobei die Gehaltsbestimmung vorzugsweise gaschromatographisch erfolgt.

Mit dem erfindungsgemässen Verfahren gelingt es, HE-HTMP aus TAA ohne Zwischenisolierung des HTMP in hohen Ausbeuten zu erhalten. Das Verfahren ist einfach durchzuführen, zeitsparend und darüber hinaus ökologisch günstig, da die bisher beim Aussalzen bzw. Umkristallisieren anfallenden Salz- bzw. Lösungsmittelmengen vermieden werden.

Die folgenden Beispiele erläutern die Erfindung näher. Teile und Prozente beziehen sich in der gesamten Beschreibung und in den Patentansprüchen auf das Gewicht, sofern nichts anderes angegeben ist. Die Gehaltsbestimmung an HTMP und HE-HTMP in den Beispielen erfolgt gaschromatographisch.

Beispiel 1: 1800 kg destilliertes TAA (Reinheitsgrad 92-98 %) werden mit 2200 Liter Wasser verdünnt und bei 70° bis 80°C und 10 bar Wasserstoffdruck in Gegenwart von Rutheniumkohle hydriert. Der Katalysator wird anschliessend abfiltriert und die HTMP-Rohlösung vor der Weiterverarbeitung in einem Tank aufbewahrt.
HTMP-Gehalt der Lösung: 40 %, Ausbeute 91-97 %

Beispiel 2: 595 g der gemäss Beispiel 1 erhaltenen HTMP-Rohlösung und 11 g $H_2SO_4$ (96%ig) werden im Autoklaven bei 92°C vorgelegt und 93 g Ethylenoxid schnell zugegeben, wobei die Temperatur auf ca. 117°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 86 %

Die Mutterlauge enthält weiteres HE-HTMP, bei dessen Aufarbeitung die Gesamtausbeute auf 90 % ansteigt.

Beispiel 3: 571 g der gemäss Beispiel 1 erhaltenen HTMP-Rohlösung werden vorgelegt und im Vakuum (500-900 mbar) bei ca. 60°C 14,5 Gew.-% der eingesetzten Lösung azeotrop abdestilliert. Es verbleibt ein Rückstand von 488 g destillativ behandelter Lösung (HTMP-Gehalt: ca. 48 Gew.-%), der zusammen mit 5 g 96%iger $HSo_4$ bei 92°C im Autoklaven vorgelegt wird. Es werden 88 g Ethylenoxid schnell zugegeben, wobei die Temperatur auf ca. 117°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 91 %

Die Mutterlauge enthält weiteres HE-HTMP, bei dessen Isolierung die Gesamtausbeute auf 97 % ansteigt.

Beispiel 4: 681 g der gemäss Beispiel 1 erhaltenen HTMP-Rohlösung werden vorgelegt und im Vakuum (500-900 mbar) bei ca. 60-80°C 30,8 Gew.-% der eingesetzten Lösung azeotrop abdestilliert. Es verbleibt ein Rückstand von 471 g destillativ behandelter Lösung (HTMP-Gehalt ca. 60 Gew.-%), der zusammen mit 3 g 96%iger $H_2SO_4$ bei 93°C im Autoklaven vorgelegt wird. Es werden 88 g Ethylenoxid schnell zugegeben, wobei die Temperatur auf ca. 119°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 92,5 %.

Die Mutterlauge enthält weiteres HE-HTMP, bei dessen Isolierung die Gesamtausbeute auf 95,3 % ansteigt.

Beispiel 5: 1663 g der gemäss Beispiel 1 erhaltenen HTMP-Rohlösung werden vorgelegt und im Vakuum (500-900 mbar) bei ca. 60-80°C 49,4 Gew.-% der eingesetzten Lösung azeotrop abdestilliert. Es verbleibt ein Rückstand von 841 g destillativ behandelter Lösung (HTMP-Gehalt: ca. 80 Gew.-%), der zusammen mit 4,3 g 96%iger $H_2SO_4$ bei 93°C im Autoklaven vorgelegt wird. Es werden 205 g Ethylenoxid schnell zugegeben, wobei die Temperatur auf ca. 138°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 93,5 %.

Die Mutterlauge enthält weiteres HE-HTMP, bei dessen Isolierung die Gesamtausbeute auf 95 % ansteigt.

Beispiel 6:1577 g der gemäss Beispiel 1 erhaltenen HTMP-Rohlösung werden vorgelegt und durch Destillation bei 80-150°C bis zur HTMP-Schmelze eingeengt. Der verbleibende Destillationsrückstand wird zusammen mit 9,5 g 96%iger $H_2SO_4$ bei 139°C

im Autoklaven vorgelegt. Es werden 195 g Ethylenoxid schnell zugegeben, wobei die Temperatur auf 165°C ansteigt (quasi-adiabatische Reaktionsführung). Nach 3 h Gesamtreaktionszeit werden 250 g Wasser zugegeben, das Reaktionsgemisch wird heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 98,3 %

Die Mutterlauge enthält weiteres HE-HTMP, bei dessen Isolierung die Gesamtausbeute aus 99,4 % ansteigt.

Beispiel 7: 579 g der gemäss Beispiel 1 erhaltenen HTMP-Lösung werden vorgelegt und im Vakuum (500-900 mbar) bei ca. 60-80°C 10 Gew.-% der eingesetzten Lösung azeotrop abdestilliert. Der verbleibende Destillationsrückstand wird zusammen mit 5,0 g 36%iger HCl bei 90°C im Autoklaven vorgelegt. Es werden 89 g Ethylenoxid schnell zugegeben, wobei die Temperatur auf ca. 110°C ansteigt. Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 90,8 %.

Die Mutterlauge enthält weiteres HE-HTMP, bei dessen Isolierung die Gesamtausbeute auf 93,6 % ansteigt.

Beispiel 8: 609 g der nach Beispiel 1 erhaltenen HTMP-Rohlösung werden vorgelegt und im Vakuum (500-900 mbar) bei ca. 60-80°C 10 Gew.-% der eingesetzten Lösung azeotrop abdestilliert. Der verbleibende Destillationsrückstand wird zusammen mit 10,0 g Eisessig bei 90°C im Autoklaven vorgelegt. Es werden 94 g Ethylenoxid schnell zugegeben, wobei die Temperatur auf ca. 110°C ansteigt. Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 81,6 %.

Die Mutterlauge enthält weiteres HE-HTMP, bei dessen Isolierung die Gesamtausbeute auf 85,2 % ansteigt.

Beispiel 9:627 g der nach Beispiel 1 erhaltenen HTMP-Rohlösung werden vorgelegt und im Vakuum (500-900 mbar) bei ca. 60-80°C 10 Gew.-% der eingesetzten Lösung azeotrop abdestilliert. Der verbleibende Destillationsrückstand wird zusammen mit 2,5 g Ameisensäure 100%ig bei 90°C im Autoklaven vorgelegt. Es werden 92 g Ethylenoxid schnell zugegeben, wobei die Temperatur auf ca. 110°C ansteigt. Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 83,8 %.

Die Mutterlauge enthält weiteres HE-HTMP, bei dessen Isolierung die Gesamtausbeute auf 86,3 % ansteigt.

Beispiel 10: 644 g der nach Beispiel 1 erhaltenen HTMP-Rohlösung werden vorgelegt und im Vakuum (500-900 mbar) bei ca. 60-80°C 10 Gew.-% der eingesetzten Lösung azeotrop abdestilliert. Der verbleibende Destillationsrückstand wird zusammen mit 10,6 g p-Toluolsulfonsäure-Monohydrat bei 90°C im Autoklaven vorgelegt. Es werden 94 g Ethylenoxid schnell zugegeben, wobei die Temperatur auf ca. 110°C ansteigt. Nach 3 h Gesamtreaktionszeit wird das Reaktionsgemisch heiss abgelassen und unter Rühren auf 20°C abgekühlt. Die Kristalle (HE-HT-MP) werden abgesaugt und mit Wasser und Toluol gewaschen.
Schmelzpunkt: 180-182°C, Ausbeute 90,1 %.

Die Mutterlauge enthält weiteres HE-HTMP, bei dessen Isolierung die Gesamtausbeute auf 92,8 % ansteigt.

Beispiel 11: 616 g der nach Beispiel 1 erhaltenen HTMP-Rohlösung werden vorgelegt und durch Destillation bei 80-150°C bis zur HTMP-Schmelze eingeengt. Der verbleibende Destillationsrückstand wird mit 353 g Butylacetat verdünnt und nach Zugabe von 5,0 g 96%iger $H_2SO_4$ bei 96°C im Autoklaven vorgelegt. Es werden 90 g Ethylenoxid schnell zugegeben, wobei die Temperatur auf 90°C absinkt. Nach 4,7 h Gesamtreaktionszeit (100°C) wird das HE-HTMP enthaltende Reaktionsgemisch heiss abgelassen und durch Destillation vollständig eingeengt.

Beispiel 12: 511 g der nach Beispiel 1 erhaltenen HTMP-Rohlösung werden vorgelegt und durch Destillation bei 80-150°C bis zur HTMP-Schmelze eingeengt. Der verbleibende Destillationsrückstand wird mit 307 g Toluol verdünnt und nach Zugabe von 5,0 g 96%iger $H_2SO_4$ bei 140°C im Autoklaven vorgelegt. Es werden 63 g Ethylenoxid schnell zugegeben, wobei die Temperatur auf 138°C absinkt. Nach 4,3 h Gesamtreaktionszeit bei 140-150°C wird das HE-HTMP enthaltende Reaktionsgemisch nach Abkühlen auf 105°C abgelassen und durch Destillation vollständig eingeengt.

Beispiel 13: 580 g der nach Beispiel 1 erhaltenen HTMP-Rohlösung werden vorgelegt und im Vakuun (500-900 mbar) bei ca. 60-80°C 10 Gew.-% der eingesetzten Lösung azeotrop abdestilliert. Der verbleibende Destillationsrückstand wird zusammen mit 26 g Toluol und 3,5 g 96%iger $H_2SO_4$ bei 90°C im Autoklaven vorgelegt. Es werden 89 g Ethylenoxid schnell zugegeben, wobei die Temperatur auf ca. 110°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 90,0 %.

Die Mutterlauge enthält weiteres HE-HTMP, bei dessen Isolierung die Gesamtausbeute auf 92,6 % ansteigt.

Beispiel 14: 618 g der nach Beispiel 1 hergestellten HTMP-Rohlösung werden vorgelegt und durch Destillation bei 80-150°C bis zur HTMP-Schmelze eingeengt. Der verbleibende Destillationsrückstand wird mit 406 g Ethylenglykol verdünnt und nach Zugabe von 3,7 g 96%iger $H_2SO_4$ bei 100°C im Autoklaven vorgelegt. Es werden 95 g Ethylenoxid schnell zugegeben, wobei die Temperatur auf ca. 121°C ansteigt (quasi-adiabatische Reaktionsführung). Nach 3 h Gesamtreaktionszeit wird das Reaktionsgemisch heiss abgelassen und unter Rühren auf 20°C abgekühlt. Die Kristalle (HE-HTMP) werden abgesaugt und mit Wasser und Toluol gewaschen.
Schmelzpunkt: 180-182°C, Ausbeute 79,8 %.

Die Mutterlauge weiteres HE-HTMP, bei dessen Isolierung die Gesamtausbeute auf 89,6 % ansteigt.

Beispiel 15: 592 g der nach Beispiel 1 hergestellten HTMP-Rohlösung werden vorgelegt und durch Destillation bei 80-150°C bis zur HTMP-Schmelze eingeengt. Der verbleibende Destillationsrückstand wird mit 104 g i-Propanol und 54 g Wasser verdünnt und nach Zugabe von 3,5 g 96%iger $H_2SO_4$ bei 82°C im Autoklaven vorgelegt. Es werden 91 g Ethylenoxid schnell zugegeben, wobei die Temperatur auf ca. 92°C ansteigt (quasi-adiabatische Reaktionsführung). Nach 3 h Gesamtreaktionszeit wird das Reaktionsgemisch heiss abgelassen, durch Destillation eingeengt und unter Rühren auf 20°C abgekühlt. Die Kristalle (HE-HTMP) werden abgesaugt und mit Wasser und Toluol gewaschen.
Schmelzpunkt: 180-182°C, Ausbeute 85,5 %.

Die Mutterlauge enthält weiteres HE-HTMP, bei dessen Isolierung die Gesamtausbeute auf 95,2 % ansteigt.

Beispiel 16: 539 g der nach Beispiel 1 hergestellten HTMP-Rohlösung werden vorgelegt und durch Destillation bei 80-150°C bis zur HTMP-Schmelze eingeengt. Der verbleibende Destillationsrückstand wird mit 323 g Methanol verdünnt und nach Zugabe von 3,2 g 96%iger $H_2SO_4$ bei 83°C im Autoklaven vorgelegt. Es werden 83 g Ethylenoxid schnell zugegeben, wobei die Temperatur auf ca. 98°C ansteigt (quasi-adiabatische Reaktionsführung). Nach 3 h Gesamtreaktionszeit wird das Reaktionsgemisch heiss abgelassen, durch Destillation eingeengt und unter Rühren auf 20°C abgekühlt. Die Kristalle (HE-HTMP) werden abgesaugt und mit Wasser und Toluol gewaschen.
Schmelzpunkt: 180-182°C, Ausbeute 81,5 %.

Die Mutterlauge enthält weiteres HE-HTMP, bei dessen Isolierung die Gesamtausbeute auf 90,0 % ansteigt.

Beispiel 17: 410 g destilliertes TAA (Reinheitsgrad 92-98 %) werden mit 290 g Methanol verdünnt und bei 80°C und 40 bar Wasserstoffdruck in Gegenwart von Rutheniumkohle hydriert. Der Katalysator wird anschliessend abfiltriert und die HTMP-Rohlösung vor der Weiterverarbeitung in einem Tank aufbewahrt.

HTMP-Gehalt der Lösung ca. 50 %, Ausbeute 90-93 %.

Beispiel 18: 712 g der nach Beispiel 17 hergestellten HTMP-Rohlösung werden nach Zugabe von 5,3 g 96%iger $H_2SO_4$ bei 85°C im Autoklaven vorgelegt. Es werden 137 g Ethylenoxid schnell zugegeben, wobei die Temperatur auf ca. 125°C ansteigt (quasi-adiabatische Reaktionsführung). Nach 3 h Gesamtreaktionszeit wird das Reaktionsgemisch heiss abgelassen, durch Destillation eingeengt und unter Rühren auf 20°C abgekühlt. Die Kristalle (HE-HTMP) werden abgesaugt und mit Wasser und Toluol gewaschen.
Schmelzpunkt: 180-182°C, Ausbeute 80,8 %.

Die Mutterlauge enthält weiteres HE-HTMP, bei dessen Isolierung die Gesamtausbeute auf 89,6 % ansteigt.

## Patentansprüche

1. Verfahren zur Herstellung von 1-(2-Hydroxyethyl)-2,2,6,6-tetramethyl-4-hydroxy-piperidin (HE-HTMP) aus 2,2,6,6-Tetramethyl-4-oxo-piperidin (TAA) , dadurch gekennzeichnet, dass man TAA in an sich bekannter Weise mittels katalytischer Hydrierung in Wasser oder in polaren organischen Lösungsmitteln oder in deren Mischungen mit Wasser zu 2,2,6,6-Tetramethyl-4-hydroxy-piperidin (HTMP) reduziert, die anfallende HTMP-Rohlösung, als solche oder nach deren Aufkonzentrierung durch Destillation, ohne weitere Reinigung und/oder Zwischenisolierung des HTMP mit einer katalytischen Menge einer anorganischen oder organischen Säure versetzt, wobei im Falle der Aufkonzentrierung die Säurezugabe auch vor der Destillation erfolgen kann, und wobei die aufkonzentrierte Rohlösung bzw. Rohschmelze gegebenenfalls mit dem gleichen oder einem anderen Lösungsmittel wieder verdünnt werden kann, und anschliessend mit Ethylenoxid in einem molaren Ueberschuss von 1-35 % bei einer Temperatur von 60-170°C umsetzt.

2. Verfahren nach Anspruch 1, worin man die HTMP-Rohlösung als solche ohne destillative Behandlung weiterverarbeitet.

3. Verfahren nach Anspruch 1, worin man die HTMP-Rohlösung durch Destillation auf einen HTMP-Gehalt von bis zu 70 % aufkonzentriert.

4. Verfahren nach Anspruch 1, worin man die HTMP-Rohlösung durch Destillation auf einen HTMP-Gehalt von mindestens 30 %, vorzugsweise mindestens 50 %, aufkonzentriert.

5. Verfahren nach Anspruch 4, worin man bis zu einer HTMP-Rohschmelze aufkonzentriert.

6. Verfahren nach Anspruch 1, worin man die katalytische Hydrierung in wässriger Lösung durchführt, gegebenenfalls die HTMP-Rohlösung durch Destillation auf bis zu 70 % HTMP-Gehalt aufkonzentriert, das Ethylenoxid in einem molaren Ueberschuss von 10-35 % bei 60-120°C bei quasi-adiabatischer Verfahrensführung zugibt und das erhaltene Produkt in bekannter Weise isoliert und aufarbeitet.

7. Verfahren nach Anspruch 3, worin die HTMP-Rohlösung vor Zugabe der katalytischen Säuremenge 30 bis 60 % HTMP enthält.

8. Verfahren nach Anspruch 1, worin man 0,1 bis 5 %, insbesondere 0,5 bis 1,0 %, bezogen auf die gegebenenfalls aufkonzentrierte und gegebenenfalls wieder verdünnte HTMP-Rohlösung bzw. -Rohschmelze, $H_2SO_4$ als katalytische Menge Säure verwendet.

9. Verfahren nach Anspruch 1, wobei das Verfahren kontinuierlich durchgeführt wird.

10. Verfahren nach Anspruch 1, worin als polare organische Lösungsmittel für die Hydrierstufe ein- oder mehrwertige Alkohole mit 1 bis 5 C-Atomen, Etheralkohole oder deren Mischungen eingesetzt werden.

11. Verfahren nach Anspruch 1, worin neben Wasser oder/und einem polaren organischen Lösungsmittels in der Hydrierstufe zusätzlich ein nicht polares organisches Lösungsmittel mitverwendet wird.

12. Verfahren nach Anspruch 1, worin nach der (teilweisen) Entfernung des Lösungsmittels der Destillationsrückstand mit einem anderen Lösungsmittel verdünnt wird.

13. Verfahren nach Anspruch 1, worin die Säurezugabe nach einer allfälligen Destillation der HTMP-Rohlösung erfolgt.

## Claims

1. A process for the preparation of 1-(2-hydroxyethyl)4-hydroxy-2,2,6,6-tetramethylpiperidine (HE-HTMP) from 4-oxo-2,2,6,6-tetramethylpiperidine (triacetoneamine, TAA), which comprises reducing TW in a manner known per se by catalytic hydrogenation in water or in a polar organic solvent, or in a mixture thereof with water, to give 4-hydroxy-2,2,6,6-tetramethylpiperidine (HTMP), adding a catalytic amount of an inorganic or organic acid to the crude HTMP solution obtained or after concentrating the solution by distillation, without further purification and/or intermediate isolation of the HTXP, where, in the case of the concentration, the addition of acid may also take place before the distillation, and where the concentrated crude solution or crude melt may be diluted again with the same solvent or with another solvent, and subsequently reacting said solution or melt with ethylene oxide in a molar excess of 1–35% at a temperature of 60–170°C.

2. A process according to claim 1, wherein the crude HTMP solution is further processed as such without distillation.

3. A process according to claim 1, wherein the crude HTMP solution is concentrated by distillation to an HTXP content of up to 70%.

4. A process according to claim 1, wherein the crude HTMP solution is concentrated by distillation to an HTMP content of at least 30%, preferably at least 50%.

5. A process according to claim 4, wherein the HTMP is concentrated to a crude melt.

6. A process according to claim 1, which comprises carrying out the catalytic hydrogenation in aqueous solution, if desired concentrating the crude HTMP solution by distillation to an HTMP content of 70%, adding the ethylene oxide in a molar excess of 10–35% at 60–120°C under quasi-adiabatic conditions, and isolating and working up the resultant product in known manner.

7. A process according to claim 3, wherein the crude HTMP solution contains 30 to 60% of HTMP before the addition of the catalytic amount of acid.

8. A process according to claim 1, wherein 0.1 to 5%, especially 0.5 to 1.0% of $H_2SO_4$, based on the optionally concentrated and optionally once more diluted crude HTMP solution or crude HTMP melt, is used as catalytic amount of acid.

9. A process according to claim 1 which is carried out continuously.

10. A process according to claim 1, wherein the polar organic solvent employed for the hydrogenation step is monohydric or polyhydric alcohol of 1 to 5 C atoms, an ether alcohol or a mixture thereof.

11. A process according to claim 1, wherein a nonpolar solvent is concurrently employed in the hydrogenation step in addition to water and/or a polar organic solvent.

12. A process according to claim 1, wherein the distillation residue is diluted with another solvent after (partial) removal of the solvent.

13. A process according to claim 1, wherein the addition of acid takes place after optional distillation of the crude HTMP solution.

## Revendications

1. Procédé pour préparer l'(hydroxy-2 éthyl)-1 tétraméthyl-2,2,6,6 hydroxy-4 pipéridine (HE-HTMP) à partir de la tétraméthyl-2,2,6,6 oxo-4 pipéridine (TAA), procédé caractérisé en ce qu'on réduit la TAA de manière connue, par hydrogénation catalytique, dans de l'eau ou dans des solvants organiques polaires ou encore dans des mélanges de ceux-ci avec de l'eau, de manière à la convertir en tétraméthyl-2,2,6,6 hydroxy-4 pipéridine (HTMP), on ajoute à la solution brute d'HTMP obtenue, telle quelle ou après l'avoir concentrée par distillation, sans purification complémentaire et/ou isolement intermédiaire de l'HTMP, une quantité catalytique d'un acide minéral ou organique -- l'addition de l'acide pouvant également avoir lieu avant la distillation dans le cas où l'on effectue une concentration, et la solution brute concentrée ou la masse fondue brute pouvant éventuellement être à nouveau diluée avec le même solvant ou avec un autre --, et on fait ensuite réagir avec l'oxyde d'éthylène en un excès molaire de 1 à 35%, à une température de 60 à 170°C.

2. Procédé selon la revendication 1 caractérisé en ce que la solution brute d'HTMP est soumise telle quelle, sans traitement par distillation, à l'opération ultérieure.

3. Procédé selon la revendication 1 caractérisé en ce que la solution brute d'HTMP est concentrée par distillation à une teneur en HTMP pouvant aller jusqu'à 70%.

4. Procédé selon la revendication 1 caractérisé en ce que la solution brute d'HTMP est concentrée par distillation à une teneur en HTMP d'au moins 30%, de préférence d'au moins 50%.

5. Procédé selon la revendication 4 caractérisé en ce qu'on concentre jusqu'à ce qu'on obtienne une masse fondue brute d'HTMP.

6. Procédé selon la revendication 1 caractérisé en ce qu'on effectue l'hydrogénation catalytique en solution aqueuse, on concentre éventuellement la solution brute d'HTMP, par distillation, à une teneur en HTMP pouvant aller jusqu'à 70%, on ajoute l'oxyde d'éthylène en un excès molaire de 10 à 35%, à une température de 60 à 120°C, dans des conditions quasi-adabiatiques, et on procède à l'isolement et au traitement complémentaire du produit de manière connue.

7. Procédé selon la revendication 3 caractérisé en ce que la solution brute d'HTMP, avant l'addition de la quantité catalytique d'acide, contient de 30 à 60% d'HTMP.

8. Procédé selon la revendication 1 caractérisé en ce qu'on utilise, comme quantité catalytique d'acide, de 0,1 à 5% d'$H_2SO_4$, plus particulièrement de 0,5 à 1,0%, par rapport à la solution brute d'HTMP ou au mélange brut d'HTMP, éventuellement concentrés et éventuellement dilués à nouveau.

9. Procédé selon la revendication 1 caractérisé en ce qu'on opère en continu.

10. Procédé selon la revendication 1 caractérisé en ce qu'on utilise, comme solvants organiques polaires pour l'étape d'hydrogénation, des monoalcools ou des polyols contenant de 1 à 5 atomes de carbone, des éther-alcools ou leurs mélanges.

11. Procédé selon la revendication 1 caractérisé en ce qu'on utilise, dans l'étape d'hydrogénation, en plus d'eau et/ou d'un solvant organique polaire, un solvant organique non polaire.

12. Procédé selon la revendication 1 caractérisé en ce qu'après l'élimination (partielle) du solvant, on dilue le résidu de distillation par un autre solvant.

13. Procédé selon la revendication 1 caractérisé en ce que l'addition d'acide est effectuée après une éventuelle distillation de la solution brute d'HTMP.